# EUROPEAN PATENT APPLICATION

(11) **EP 1 317 942 A1**
(43) Date of publication of application: **11.06.2003**
(21) Application number: 02027282.9
(22) Date of filing: 06.12.2002
(51) Int. Cl.: A61M 16/12, F04D 25/04

(54) **Gases mixing apparatus**

(30) Priority: 07.12.2001 NZ 51600901
(71) Applicant: Fisher & Paykel Healthcare Limited, East Tamaki, Auckland (NZ)
(72) Inventor: Mackie, Scott Robert, Balmoral, Auckland (NZ)
(74) Representative: Brown, John David

(57) **Abstract**

A gas and air mixing device receives a pressurised supply of a gas, drawing an air supply from the ambient environment and outputs a pressurised combined mixture of air and said gas. The device includes a turbine (2) with a gases inlet (6) and a gases outlet (10) and a pump (4) with an air inlet (13) and an air outlet (15). A combined gases outlet (16) receives gases from the gases outlet (10) of the turbine and air from the air outlet (15) of the pump. The combined gases outlet ouputs a combined flow of the gases. The turbine is configured to drive the pump.

## Description

### BACKGROUND

### Field of the Invention

The present invention relates to devices for the mixing of air and oxygen in a medical environment.

### Summary of the Prior Art

It is often desirable to supply a patient or person with an air stream enriched in oxygen to a greater concentration than ambient air. For this purpose a supply of oxygen is mixed with a supply of air for breathing by the patient.

Where a patient is capable of normal breathing, an air entrainer may be added to a breathing intake tube. The air entrainer includes an oxygen inlet nozzle receiving a high pressure oxygen supply from a high pressure oxygen supply source. An air inlet port is provided in the wall of the entrainer adjacent the nozzle outlet. High velocity oxygen flow through the nozzle outlet and into the conduit draws air into the conduit through the air inlet port. This air becomes mixed with the oxygen flow as it passes along the conduit to the patient. The mixing ratio may be adjusted by adjusting the size of the air inlet opening. The passive air entrainer is effective where the conduit has no significant resistance to flow. The passive air entrainer is unable to operate effectively where there is a significant flow resistance.

For example, where the patient requires positive pressure therapy the air entrainer is ineffective. Positive pressure therapy is presently considered desirable in treatment in a range of respiratory ailments.

In many situations it is desirable to humidify respiratory gases provided to a patient. Many devices used to humidify such gases unavoidably add significant flow resistance to the breathing intake tube. Air entrainers that operate in the manner described above must therefore be placed between the humidification device and the patient to operate correctly.

The consequence of this placement is that the entrained ambient air does not pass through the humidifier and is not humidified.

To overcome this difficulty an alternative mixed air/oxygen supply apparatus is commonly used in which the supplied gases are drawn from a compressed air supply and a compressed oxygen supply. Each gas is supplied through a regulator valve. The relative flow rate of each gas is thereby adjustable and the flows undergo a simple combination into a supply conduit.

Alternatively a mixed air/oxygen supply apparatus known as a blender uses partial pressure to provide a pressurised source of air and oxygen, controlling the source pressure of each of the gases to be mixed. Such a device comes with a dial to set the oxygen concentration of the new pressurised source. A blender is usually connected to a regulator valve with which the user can set the desired flow rate of the blended gas.

Both these systems can provide a high pressure air/oxygen mixture which can be used to provide positive pressure therapy and is suitable to supply gases via a humidifier. The disadvantage of these systems is the need for a compressed air supply in addition to the compressed oxygen supply, and in the case of the blender, considerable cost.

### Summary of the Invention

t is an object of the present invention to provide an air/oxygen mixing device which at least goes some way towards overcoming the above disadvantages or will at least provide health providers with a useful choice.

In a first aspect the present invention may broadly be said to consist in a gas and air mixing device for receiving a pressurised supply of a gas, drawing an air supply from the ambient environment and outputting a pressurised combined mixture of air and said gas comprising:
a turbine having a gases inlet and a gases outlet,
a pump having an air inlet and an air outlet, and
a combined gases outlet receiving gases from said gases outlet of said turbine and air from said air outlet of said pump and outputting a combined flow;
said turbine being configured to drive said pump.

Preferably said turbine comprises:
a first chamber having an oxygen inlet and an oxygen outlet and a turbine in said first chamber receiving oxygen from said oxygen inlet;
said pump comprises:
   a second chamber having an air inlet and an air outlet, and
   an impeller in said second chamber receiving air from said air inlet; and
   a drive train connects between said turbine and said impeller.

Preferably each of the components is formed from medical grade plastics, for example by injection moulding. Some forms of turbine in particular may be difficult to form by injection moulding in one piece and may be instead formed as two pieces combined by ultrasonic welding, fasteners, adhesive bonding, integral clipping or other equivalent arrangements. Some other parts may require strength or other properties not obtainable with plastic and may be formed from another material such as aluminium, magnesium or stainless steel.

Preferably the oxygen inlet is configured for connection with an oxygen wall outlet or a compressed oxygen bottle outlet.

Preferably said gas inlets and outlets may include adjustable flow dampers.

Preferably said adjustable flow dampers include a calibration system corresponding with the proportion of air or oxygen in the mixed flow for at least one set oxygen inlet pressure.

Preferably said first chamber and said second chamber are back to back, said turbine and said impeller are co axial, and said drive train comprises a shaft, and both said turbine and said impeller are connected to said shaft.

Preferably said shaft is an integrally formed extension from the centre of said impeller, extending through a dividing wall between said first chamber and said second chamber, into said first chamber, and said turbine is mounted on said extension.

Preferably said first chamber is formed by a cover member fitted to said dividing wall enclosing said turbine.

Preferably said second chamber is formed from a cover fitted to said dividing wall and enclosing said impeller, said second cover including an air inlet at the centre of its main face.

Alternatively said device is arranged axially, said turbine comprising
an axial turbine having a gases inlet and gases outlet,
said pump comprising an axial pump receiving gases from said gases outlet of said turbine and air from a separate air inlet, and
said combined gases outlet receiving gases from said pump and outputting a combined flow;
with said turbine and pump being arranged in axial alignment and said turbine being configured to drive said pump.

Preferably said device includes a housing with a first section and a second section, said first section having said oxygen inlet and said oxygen outlet and housing a rotor of said turbine; and
said second section having an air inlet, an oxygen inlet and said pump gases outlet, and
housing an impeller;
a drive train connecting between said rotor and said impeller.

Preferably each of the components is formed from medical grade plastics, for example by injection moulding. Some forms of turbine in particular may be difficult to form by injection moulding in one piece and may be instead formed as two pieces combined by ultrasonic welding, fasteners, adhesive bonding, integral clipping or other equivalent arrangements. Some other parts may require strength or other properties not obtainable with plastic and may be formed from another material such as aluminium, magnesium or stainless steel.

Preferably the oxygen inlet of said first section is configured for connection with an oxygen wall outlet or a compressed oxygen bottle outlet.

Preferably said gas inlets and outlets may include adjustable flow dampers.

Preferably said adjustable flow dampers include a calibration system corresponding with the proportion of air or oxygen in the mixed flow for at least one set oxygen inlet pressure.

Preferably said first section and said second section are coaxial and concentric over at least part of their length, said turbine rotor and said impeller are co-axial, and said drive train comprises a shaft, and both said turbine and said impeller are connected to said shaft.

Preferably said shaft is an integrally formed extension from the centre of said impeller, mounted concentrically within said first and second sections

Preferably said first and second sections are connected in a manner which allows unrestricted flow of ambient air into said second section whilst remaining structurally sufficient

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting.

### BRIEF DESCRIPTION OF THE DRAWINGS

Figure 1 is a cross sectional front elevation through an air oxygen mixer according to the present invention.
Figure 2 is a front elevation of the air/oxygen mixer of Figure 1.
Figure 3 is a side elevation of the air/oxygen mixer of Figure 1.
Figure 4 is a perspective exploded view of the air/oxygen mixer of Figure 1.
Figure 5 is a top view of the mixer of Figure 1 as connected in use including the combined flow outlet and the oxygen inlet connection.
Figure 6 is a perspective view of the air inlet portion of a second chamber housing according to a preferred embodiment of the present invention with partial cutaway.
Figure 7 is a perspective exploded view of an air/oxygen mixer according to another embodiment of the present invention.
Figure 8 is a cross sectional exploded view of the air/oxygen mixer of Figure 7.
Figure 9 is a perspective view of the assembled air/oxygen mixer of Figure 7.

### DETAILED DESCRIPTION

One preferred embodiment of the present invention is disclosed in the Figures. It will be appreciated that significant variations may be applied to this preferred embodiment without departing from the scope of the present invention.

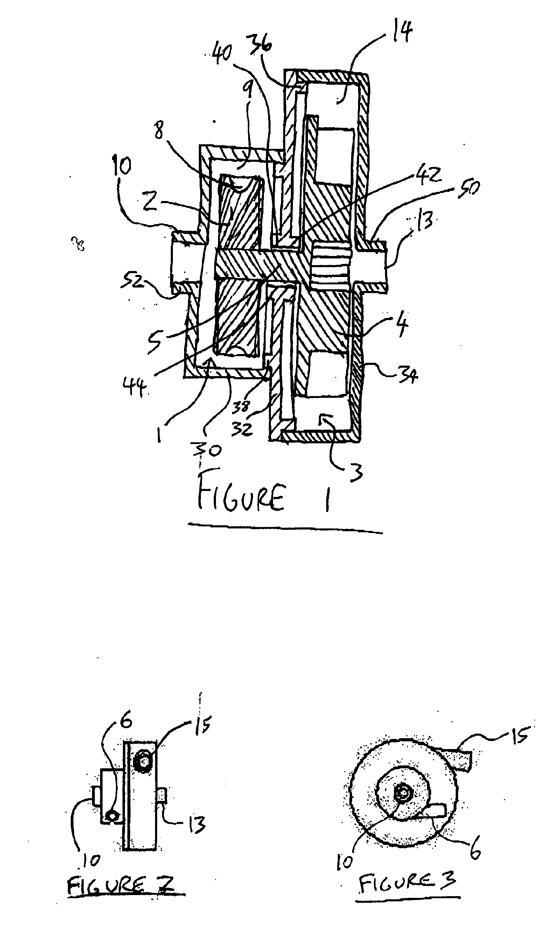

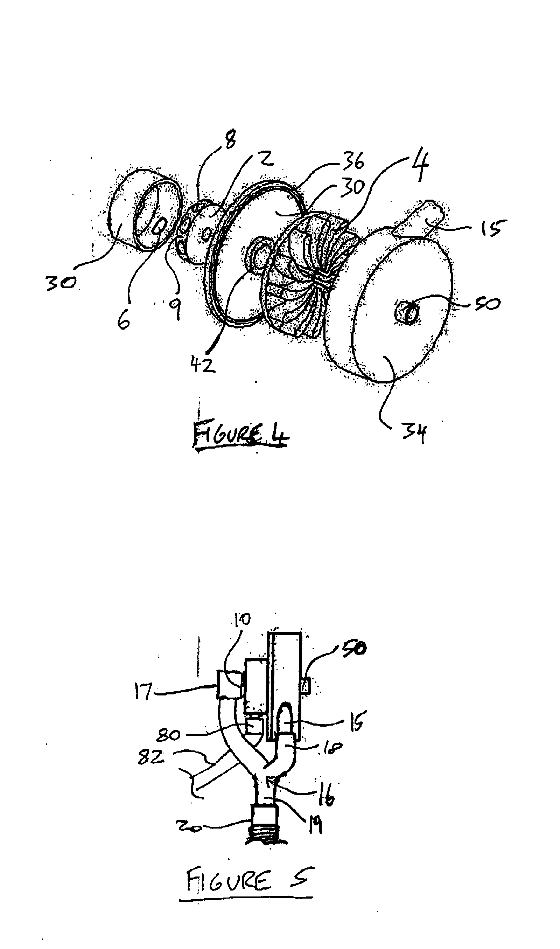

Referring in particular to Figures 1, 2 and 5 the oxygen mixing device according to the preferred embodiment includes a first chamber 1 enclosing a turbine rotor 2 and a second chamber 3 enclosing an impeller 4. A shaft 5 extends from the impeller 4 into the first chamber 1. The turbine rotor 2 is mounted on the shaft 5.

In one preferred form, the turbine is of a Pelton wheel type, and the rotor 2 includes a plurality of peripheral cups formed as depressions 8 in its annular face 9. Compressed oxygen is supplied through an oxygen inlet 6. The compressed oxygen leaves the oxygen inlet 6 as a jet and impacts the cups of turbine rotor 2, driving rotation of turbine rotor2. The air exits the turbine enclosure 1 through an oxygen outlet 10. Rotation of the rotor 2 rotates shaft 5 and impeller 4. The impeller 4 is a centrifugal air pump impeller. Rotation of impeller 4 draws air through an air inlet 13 adjacent the centre of impeller 4. The air is spun outwards between the impeller blades to a peripheral exit zone 14 and exits by exit 15.

A flow combining connector is connected with the oxygen outlet 10 and the air outlet 15. The flow combining connector comprises for example a flexible tube Y-piece 16 with a first end 17 fitted over oxygen outlet 10 and a second end 18 fitted over air outlet 15, the flows combining through a third leg 19.

In the preferred embodiment of the invention the first chamber 1 is formed between a first cover 30 and a dividing wall member 32 and the second chamber 3 is enclosed between a second cover 34 and the dividing wall member 32. The dividing wall member 32 is a substantially circular disk and includes an annular flange 38 extending from one face to support the rim of cover 30 and an annular flange 36 extending from its other face to support the rim of cover 34. The dividing member 32 includes a central aperture 40 receiving the shaft 5 therethrough. An annular flange 42 surrounding aperture 40 extends from the air chamber surface of dividing member 32, and a similar annular flange 44 extends from the oxygen chamber face of member 32. The flanges provide additional bearing support for the shaft 5 and thrust bearing surfaces for the impeller 4 and rotor 2. Additional bearings may be used between the flanges 42 and 44 and the shaft 5 to provide a longer life product. The air chamber cover 34 includes air inlet 13 as an open short tube 50 extending from the centre of its main face. The oxygen chamber cover 30 includes oxygen outlet 6 as an open short tube 52 extending from the centre of its main face.

Accordingly, in the preferred embodiment of the present invention the device is comprised of six components - the first cover 30, the second cover 34, the dividing wall member 32, the impeller (with integral shaft 5), the turbine 2 and the flow combining connector 16.

It should be appreciated that substantial variation in the actual construction is possible without departing from the scope of the invention, and advantageous variations may include integrating the flow combining connector 16 with the covers 30 and 34 (for example by providing the outlet passages 10 and 15 as a combination of the cover 30 or 34 respectively and the dividing wall 32, and leading to a common location to combine as a single outlet connector). Other variations may include using other forms of turbine for extracting energy from the high pressure oxygen flow, and/or other forms of impeller for creating the air flow.

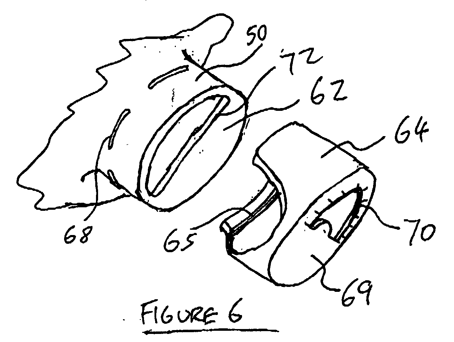

Another modification may include the provision of an inlet air damper on air inlet 13. An example of an appropriate adapter is depicted in Figure 6 where air inlet tube 13 is partially enclosed by covering face end 62. A cap 64 is fitted over the inlet tube 50, and retained in place by channel 55 fitting over protrusions 68 from the cylindrical surface of inlet tube 50. The cap 64 includes a cylindrical body and an enclosed end 69. The enclosed end 69 includes a tapering aperture 70 with a graduated scale marked around its outer perimeter.

In use the orientation of the cap 64 on the inlet tube 50 determines the size of the inlet opening by the intersection between the aperture 70 and the uncovered portion of the end of tube 50. The graduated scale of aperture 70 may be calibrated for a set oxygen inlet pressure with a scale corresponding to the proportion of air resulting in the air/oxygen mix. The indicator relative to the scale may be the edge 72 of the covered in portion 62 of the end of tube 50. The cap 64 is rotatable on the tube 50 by sliding of protrusions 68 within channel 65 to vary the amount of opening of the air inlet.

The device is illustrated in use in Figure 5. A compressed oxygen supply tube 82 is connected with the short oxygen supply tube of housing 30 via a connector 80. The flow combining connector 16 is connected with a breathing tube 20. When the compressed oxygen supply is started this spins turbine rotor 2 in its passage through the first chamber to outlet 10. Rotor 2 spins impeller 4 causing the impeller 4 to draw air into the second chamber 3 through inlet 50 and expel the air through outlet 15. The outlet flows combine in connector 16 and exit to breathing conduit 20 under pressure.

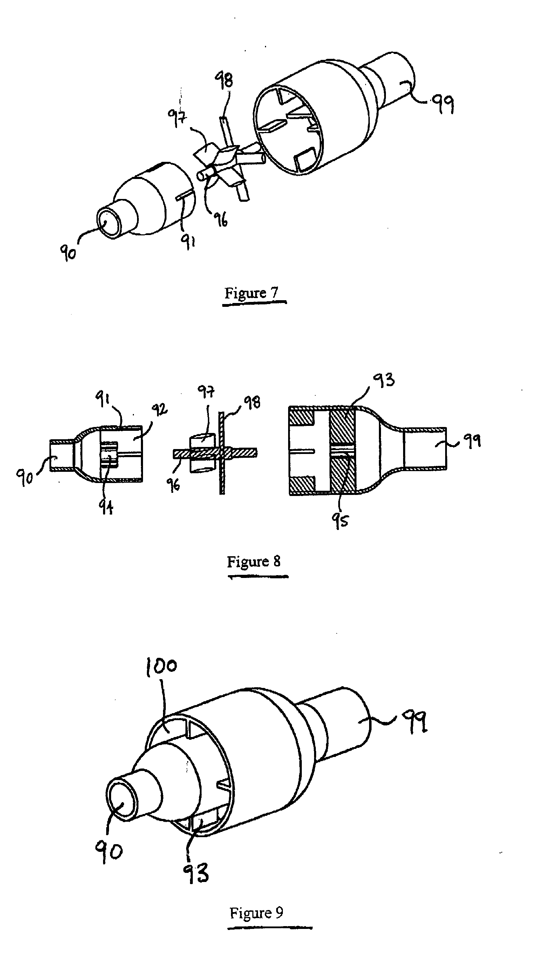

A second preferred embodiment of the present invention is disclosed in Figures 7, 8 and 9. It will be appreciated that significant variations may be also be applied to this second preferred embodiment without departing from the scope of the present invention.

The second embodiment is an axial arrangement. It includes an axial flow turbine and an axial flow pump. The axial flow turbine and pump are arranged end to end. The turbine includes a rotor or impeller 97, and the pump includes an impeller 98, with the turbine rotor 97 arranged to drive the pump impeller 98.

The turbine impeller 97 and the pump impeller 98 are located on a common shaft. A first housing surrounds the turbine impeller 97. The first housing includes an oxygen inlet 90 and an oxygen outlet 92. A second housing is coaxial with the first housing, and is concentric with the first housing over a part of their length where the two housings overlap. The second housing is greater diameter than the first housing at this overlap. The second housing thus has, centrally, an inlet for oxygen that has passed through the turbine, and an annular air inlet 100 surrounding the outlet end of the first housing.

The shaft 96 is supported so as not to impede flow either side of the impellers. Struts 92 protuding from the inner surface of the oxygen inlet casing support the shaft at the turbine end and similar struts 93 at the fan end. These struts can be shaped aerodynamically to reduce resistance to flow past them and to have positive effects on flow characteristics either side of the impellers.

High pressure oxygen flowing through the oxygen inlet must pass by the axial turbine impeller 97 thereby imparting a force that will rotate the shaft. This forced rotation is transmitted to the axial fan impeller 98. The rotation of the fan impeller, which is larger diameter than the outlet end of the first housing, sucks ambient air through the air inlet 100 where it becomes mixed with oxygen that has passed through both the turbine and fan blades. The mixture of gases is then driven through gases outlet 99 by the pressure created by the fan impeller 98.

So for example the device may receive an oxygen supply at 50 psi above ambient pressure and an air supply at ambient pressure, receiving approximately 20 litres of oxygen per minute and extracting energy from the oxygen flow to drive the air pump. In turn the air pump sucks in the ambient air and supplies it at its outlet at for example 2 psi above ambient pressure and at a flow of 20 litres per minute, providing a combined flow from outlet connector 99 of 40 litres per minute at 2 psi static pressure at approximately 60% Oxygen.

It will therefore be appreciated that the oxygen supply side remains under pressure from its supply source and the air supply side is positively driven by the pump impeller. This makes the device less susceptible to circuit back pressures than a passive air entrainer, while eliminating the need for a compressed air supply.

The features disclosed in the foregoing description, in the claims and/or in the accompanying drawings may, both separately and in any combination thereof, be material for realising the invention in diverse forms thereof.

## Claims

1. A gas and air mixing device for receiving a pressurised supply of a gas, drawing an air supply from the ambient environment and outputting a pressurised combined mixture of air and said gas comprising:
a turbine having a gases inlet and a gases outlet,
a pump having an air inlet and an air outlet, and
a combined gases outlet receiving gases from said gases outlet of said turbine and air from said air outlet of said pump and outputting a combined flow;
said turbine being configured to drive said pump.

2. A mixing device as claimed in claim 1 wherein,
said turbine comprises:
a first chamber having an oxygen inlet and an oxygen outlet and a turbine in
said first chamber receiving oxygen from said oxygen inlet;
said pump comprises:
a second chamber having an air inlet and an air outlet, and
an impeller in said second chamber receiving air from said air inlet; and
a drive train connects between said turbine and said impeller.

3. A mixing device as claimed in claim 2 wherein the oxygen inlet is configured for connection with an oxygen wall outlet or a compressed oxygen bottle outlet.

4. A mixing device as claimed in claim 2 wherein said air inlet includes an adjustable air flow damper.

5. A mixing device as claimed in claim 4 wherein said adjustable air flow damper includes a calibration corresponding with the proportion of air or oxygen in the mixed flow for at least one set oxygen inlet pressure.

6. A mixing device as claimed in claim 2 wherein said first chamber and said second chamber are back to back, said turbine and said impeller are co axial, and said drive train comprises a shaft, and both said turbine and said impeller are connected to said shaft.

7. A mixing device as claimed in claim 6 wherein said shaft is an integrally formed extension from the centre of said impeller, extending through a dividing wall between said first chamber and said second chamber, into said first chamber, and said turbine is mounted on said extension.

8. A mixing device as claimed in claim 7 wherein said first chamber is formed by a cover member fitted to said dividing wall enclosing said turbine.

9. A mixing device as claimed in claim 8 wherein said second chamber is formed from a cover fitted to said dividing wall and enclosing said impeller, said second cover including an air inlet at the centre of its main face.

10. A mixing device as claimed in claim 1 wherein said device is arranged axially, said turbine comprising
an axial turbine having a gases inlet and gases outlet,
said pump comprising an axial pump receiving gases from said gases outlet of said turbine and air from a separate air inlet, and
said combined gases outlet receiving gases from said pump and outputting a combined flow;
with said turbine and pump being arranged in axial alignment and said turbine being configured to drive said pump.

11. A mixing device as claimed in claim 10 wherein said device includes a housing with a first section and a second section, said first section having said oxygen inlet and said oxygen outlet and housing a rotor of said turbine; and
said second section having an air inlet, an oxygen inlet and said pump gases outlet, and
housing an impeller;
a drive train connecting between said rotor and said impeller.

12. A mixing device as claimed in claim 10 wherein the oxygen inlet is configured for connection with an oxygen wall outlet or a compressed oxygen bottle outlet.

13. A mixing device as claimed in claim 10 wherein said air inlet includes an adjustable air flow damper.

14. A mixing device as claimed in claim 13 wherein said adjustable air flow damper includes a calibration corresponding with the proportion of air or oxygen in the mixed flow for at least one set oxygen inlet pressure.

15. A mixing device as claimed in claim 11 wherein said first section and said second section are coaxial and concentric over at least part of their length, said turbine rotor and said impeller are co-axial, and said drive train comprises a shaft, and both said turbine and said impeller are connected to said shaft.

16. A mixing device as claimed in claim 15 wherein said shaft is an integrally formed extension from the centre of said impeller, mounted concentrically within said first and second sections

17. A mixing device as claimed in claim 16 wherein said first and second sections are connected in a manner which allows unrestricted flow of ambient air into said second section through an annular opening between overlapping portions of said first section and said second section.
